# EUROPEAN PATENT SPECIFICATION

(11) **EP 3 402 505 B1**
(45) Date of publication and mention of the grant of the patent: **17.06.2020**
(21) Application number: 17701799.3
(22) Date of filing: 11.01.2017
(51) Int. Cl.: A61K 38/08, A61K 38/07, A61K 38/09, A61K 38/28, A61K 9/00, C07K 5/11, C07K 5/113, C07K 7/06, A61P 31/10

(54) **PHARMACEUTICAL FORMULATIONS FOR THE TREATMENT OF DIABETES**
PHARMAZEUTISCHE FORMULIERUNGEN ZUR BEHANDLUNG VON DIABETES
FORMULATIONS PHARMACEUTIQUES POUR LE TRAITEMENT DU DIABÈTE

(30) Priority: 12.01.2016 IT UB20169928
(43) Date of publication of application: 21.11.2018
(73) Proprietor: Kaleyde Pharmaceuticals AG, 6900 Lugano (CH)
(72) Inventor: DE ROSA, Mario, 80131 Napoli (IT)
(74) Representative: Bianchetti Bracco Minoja S.r.l.
(86) International application number: PCT/EP2017/050495
(87) International publication number: WO 2017/121764

(56) References cited:
- WO-A1-01/72770
- WO-A1-2008/017372
- WO-A2-02/060473
- WO-A2-2004/087206

### SEQUENCE LISTING

<110> 1695PCT
<120> Pharmaceutical formulations for the treatment of diabetes
<130> MEDICAL AND BIOTECHNOLOGICAL SERVICES IN SIGLA M.B.S. S.R.L.
<160> 65
<170> PatentIn version 3.5
<210> 1
   <211> 5
   <212> PRT
   <213> Artificial Sequence
<220>
   <223> synthetic peptide
<220>
   <221> MISC_FEATURE
   <222> (1)..(5)
<220>
   <221> MOD_RES
   <222> (5)..(5)
   <223> AMIDATION
<400> 1
   Glu Arg Glu Arg Phe
   1               5
<210> 2
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (4)..(4)
   <223> AMIDATION
<400> 2
   Arg Glu Arg Tyr
   1
<210> 3
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (4).. (4)
   <223> AMIDATION
<400> 3
   Arg Glu Arg Trp
   1
<210> 4
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (3)..(3)
   <223> X is N(Me) Arg
<220>
   <221> MOD_RES
   <222> (4)..(4)
   <223> AMIDATION
<400> 4
   Arg Glu Xaa Phe
   1
<210> 5
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (3)..(3)
   <223> X is N(Me)Arg
<220>
   <221> MOD_RES
   <222> (4)..(4)
   <223> AMIDATION
<400> 5
   Arg Glu Xaa Tyr
   1
<210> 6
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (3)..(3)
   <223> X is N(Me)Arg
<220>
   <221> MOD_RES
   <222> (4)..(4)
   <223> AMIDATION
<400> 6
   Arg Glu Xaa Trp
   1
<210> 7
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MISC_FEATURE
   <222> (1)..(5)
<220>
   <221> MOD_RES
   <222> (4).. (4)
   <223> X is N(Me)Arg
<220>
   <221> MOD_RES
   <222> (5)..(5)
   <223> AMIDATION
<400> 7
   Xaa Arg Glu Xaa Phe
   1               5
<210> 8
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MISC_FEATURE
   <222> (1)..(5)
<220>
   <221> MOD_RES
   <222> (4).. (4)
   <223> X is N(Me)Arg
<220>
   <221> MOD_RES
   <222> (5)..(5)
   <223> AMIDATION
<400> 8
   Xaa Arg Glu Xaa Tyr
   1               5
<210> 9
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MISC_FEATURE
   <222> (1)..(1)
<220>
   <221> MISC_FEATURE
   <222> (4)..(4)
<220>
   <221> MOD_RES
   <222> (4)..(4)
   <223> Xis N(Me)Arg
<220>
   <221> MOD_RES
   <222> (5)..(5)
   <223> AMIDATION
<400> 9
   Xaa Arg Glu Xaa Trp
   1               5
<210> 10
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MOD_RES
   <222> (5)..(5)
   <223> AMIDATION
<400> 10
   Xaa Arg Glu Arg Phe
   1               5
<210> 11
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MOD_RES
   <222> (5)..(5)
   <223> AMIDATION
<400> 11
   Xaa Arg Glu Arg Tyr
   1               5
<210> 12
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MOD_RES
   <222> (5)..(5)
   <223> AMIDATION
<400> 12
   Xaa Arg Glu Arg Trp
   1               5
<210> 13
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<400> 13
   Arg Xaa Arg Phe
   1
<210> 14
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<220>
   <221> MOD_RES
   <222> (4)..(4)
   <223> AMIDATION
<400> 14
   Arg Xaa Arg Tyr
   1
<210> 15
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<220>
   <221> MOD_RES
   <222> (4)..(4)
   <223> AMIDATION
<400> 15
   Arg Xaa Arg Trp
   1
<210> 16
   <211> 5
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> Aib
<220>
   <221> MOD_RES
   <222> (3)..(3)
   <223> Aib
<220>
   <221> MOD_RES
   <222> (5)..(5)
   <223> AMIDATION
<400> 16
   Xaa Arg Xaa Arg Phe
   1               5
<210> 17
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<220>
   <221> MOD_RES
   <222> (4)..(4)
   <223> AMIDATION
<400> 17
   Arg Xaa Arg Phe
   1
<210> 18
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<220>
   <221> MOD_RES
   <222> (3)..(3)
   <223> X is N(Me)Arg
<220>
   <221> MOD_RES
   <222> (4)..(4)
   <223> AMIDATION
<400> 18
   Arg Xaa Xaa Tyr
   1
<210> 19
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<220>
   <221> MOD_RES
   <222> (3)..(3)
   <223> X is N(Me)Arg
<220>
   <221> MOD_RES
   <222> (4)..(4)
   <223> AMIDATION
<400> 19
   Arg Xaa Xaa Trp
   1
<210> 20
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MOD_RES
   <222> (3)..(3)
   <223> Aib
<220>
   <221> MOD_RES
   <222> (4)..(4)
   <223> X is N(Me)Arg
<220>
   <221> MOD_RES
   <222> (5)..(5)
   <223> AMIDATION
<400> 20
   Xaa Arg Xaa Xaa Phe
   1               5
<210> 21
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MOD_RES
   <222> (3)..(3)
   <223> Aib
<220>
   <221> MOD_RES
   <222> (4)..(4)
   <223> X is N(Me)Arg
<220>
   <221> MOD_RES
   <222> (5)..(5)
   <223> AMIDATION
<400> 21
   Xaa Arg Xaa Xaa Tyr
   1               5
<210> 22
   <211> 5
   <212> PRT
   <213> Artificial Sequence
<220>
   <223> synthetic peptide
<220>
   <221> MOD_RES
   <222> (1)..(1)
   <223> X is pGlu
<220>
   <221> MOD_RES
   <222> (3)..(3)
   <223> Aib
<220>
   <221> MOD_RES
   <222> (4)..(4)
   <223> X is N(Me)Aeg
<220>
   <221> MOD_RES
   <222> (5)..(5)
   <223> AMIDATION
<400> 22
   Xaa Arg Xaa Xaa Trp
   1               5
<210> 23
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Aib
<220>
   <221> MOD_RES
   <222> (5)..(5)
   <223> AMIDATION
<400> 23
   Xaa Arg Xaa Arg Phe
   1               5
<210> 24
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Aib
<220>
   <221> MOD_RES
   <222> (5)..(5)
   <223> AMIDATION
<400> 24
   Xaa Arg Xaa Arg Tyr
   1               5
<210> 25
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Aib
<220>
   <221> MOD_RES
   <222> (5)..(5)
   <223> AMIDATION
<400> 25
   Xaa Arg Xaa Arg Trp
   1               5
<210> 26
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2).. (2)
   <223> Ac5c
<220>
   <221> MOD_RES
   <222> (4)..(4)
   <223> AMIDATION
<400> 26
   Arg Xaa Arg Phe
   1
<210> 27
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2).. (2)
   <223> Ac5c
<220>
   <221> MOD_RES
   <222> (4).. (4)
   <223> AMIDATION
<400> 27
   Arg Xaa Arg Tyr
   1
<210> 28
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2).. (2)
   <223> Ac5c
<220>
   <221> MOD_RES
   <222> (4)..(4)
   <223> AMIDATION
<400> 28
   Arg Xaa Arg Trp
   1
<210> 29
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Ac5c
<220>
   <221> MOD_RES
   <222> (3)..(3)
   <223> N(Me)Arg
<220>
   <221> MOD_RES
   <222> (4)..(4)
   <223> AMIDATION
<400> 29
   Arg Xaa Xaa Phe
   1
<210> 30
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Ac5c
<220>
   <221> MOD_RES
   <222> (3)..(3)
   <223> N(Me)Arg
<220>
   <221> MOD_RES
   <222> (4)..(4)
   <223> AMIDATION
<400> 30
   Arg Xaa Xaa Tyr
   1
<210> 31
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Ac5c
<220>
   <221> MOD_RES
   <222> (3)..(3)
   <223> N(Me)Arg
<220>
   <221> MOD_RES
   <222> (4)..(4)
   <223> AMIDATION
<400> 31
   Arg Xaa Xaa Trp
   1
<210> 32
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Ac5c
<220>
   <221> MOD_RES
   <222> (4)..(4)
   <223> N(Me)Arg
<220>
   <221> MOD_RES
   <222> (5)..(5)
   <223> AMIDATION
<400> 32
   Xaa Arg Xaa Xaa Phe
   1               5
<210> 33
   <211> 5
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> ACETYLATION
<220>
   <221> MOD_RES
   <222> (1)..(1)
   <223> Xaa is pGlu
<220>
   <221> MOD_RES
   <222> (3)..(3)
   <223> Ac5c
<220>
   <221> MOD_RES
   <222> (4).. (4)
   <223> N(Me)Arg
<220>
   <221> MOD_RES
   <222> (5)..(5)
   <223> AMIDATION
<400> 33
   Xaa Arg Xaa Xaa Tyr
   1               5
<210> 34
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Ac5c
<220>
   <221> MOD_RES
   <222> (4).. (4)
   <223> N(Me)Arg
<220>
   <221> MOD_RES
   <222> (5)..(5)
   <223> AMIDATION
<400> 34
   Xaa Arg Xaa Xaa Trp
   1               5
<210> 35
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Ac5c
<220>
   <221> MOD_RES
   <222> (5)..(5)
   <223> AMIDATION
<400> 35
   Xaa Arg Xaa Arg Phe
   1               5
<210> 36
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Ac5c
<220>
   <221> MOD_RES
   <222> (5)..(5)
   <223> AMIDATION
<400> 36
   Xaa Arg Xaa Arg Tyr
   1               5
<210> 37
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Ac5c
<220>
   <221> MOD_RES
   <222> (5)..(5)
   <223> AMIDATION
<400> 37
   Xaa Arg Xaa Arg Trp
   1               5
<210> 38
   <211> 4
   <212> PRT
   <213> Artificial Sequence
<220>
   <223> synthetic peptide
<220>
   <221> MOD_RES
   <222> (1)..(1)
   <223> ACETYLATION
<400> 38
   Arg Glu Arg Phe
   1
<210> 39
   <211> 4
   <212> PRT
   <213> Artificial Sequence
<220>
   <223> synthetic peptide
<220>
   <221> MOD_RES
   <222> (1)..(1)
   <223> ACETYLATION
<400> 39
   Arg Glu Arg Tyr
   1
<210> 40
   <211> 4
   <212> PRT
   <213> Artificial Sequence
<220>
   <223> synthetic peptide
<220>
   <221> MOD_RES
   <222> (1)..(1)
   <223> ACETYLATION
<400> 40
   Arg Glu Arg Trp
   1
<210> 41
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (3)..(3)
   <223> N(Me)Arg
<400> 41
   Arg Glu Xaa Tyr
   1
<210> 42
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (4)..(4)
   <223> Arg(Me)
<400> 42
   Xaa Arg Glu Xaa Phe
   1 5
<210> 43
   <211> 5
   <212> PRT
   <213> Artificial Sequence
<220>
   <223> synthetic peptide
<220>
   <221> MOD_RES
   <222> (1)..(1)
   <223> pGlu
<400> 43
   Xaa Arg Glu Arg Trp
   1 5
<210> 44
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2).. (2)
   <223> Aib
<220>
   <221> MOD_RES
   <222> (3)..(3)
   <223> Arg(Me)
<400> 44
   Arg Xaa Xaa Phe
   1
<210> 45
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2).. (2)
   <223> Aib
<220>
   <221> MOD_RES
   <222> (3)..(3)
   <223> Arg(Me)
<400> 45
   Arg Xaa Xaa Phe
   1
<210> 46
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Aib
<220>
   <221> MOD_RES
   <222> (4).. (4)
   <223> Arg(Me)
<400> 46
   Xaa Arg Xaa Xaa Tyr
   1               5
<210> 47
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Aib
<400> 47
   Xaa Arg Xaa Arg Trp
   1               5
<210> 48
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2).. (2)
   <223> Ac5c
<400> 48
   Arg Xaa Arg Phe
   1
<210> 49
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2).. (2)
   <223> Ac5c
<220>
   <221> MOD_RES
   <222> (3)..(3)
   <223> Arg(Me)
<400> 49
   Arg Xaa Xaa Tyr
   1
<210> 50
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Ac5c
<220>
   <221> MOD_RES
   <222> (4)..(4)
   <223> Arg(Me)
<400> 50
   Xaa Arg Xaa Xaa Trp
   1 5
<210> 51
   <211> 5
   <212> PRT
   <213> Artificial Sequence
<220>
   <223> synthetic peptide
<220>
   <221> MOD_RES
   <222> (1)..(1)
   <223> pGlu
<220>
   <221> MOD_RES
   <222> (3)..(3)
   <223> Ac5c
<400> 51
   Xaa Arg Xaa Arg Trp
   1 5
<210> 52
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> N(Me)Arg
<220>
   <221> MOD_RES
   <222> (2)..(2)
   <223> Aib
<220>
   <221> MOD_RES
   <222> (4)..(4)
   <223> AMIDATION
<400> 52
   Xaa Xaa Arg Phe
   1
<210> 53
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> N(Me)Arg
<220>
   <221> MOD_RES
   <222> (2)..(2)
   <223> Aib
<220>
   <221> MOD_RES
   <222> (3)..(3)
   <223> N(Me)Arg
<220>
   <221> MOD_RES
   <222> (4)..(4)
   <223> AMIDATION
<400> 53
   Xaa Xaa Xaa Phe
   1
<210> 54
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<220>
   <221> MOD_RES
   <222> (3)..(3)
   <223> N(Me)Arg
<220>
   <221> MOD_RES
   <222> (4)..(4)
   <223> AMIDATION
<400> 54
   Arg Xaa Xaa Phe
   1
<210> 55
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<220>
   <221> MOD_RES
   <222> (4)..(4)
   <223> alpha(Me)Phe
<220>
   <221> MOD_RES
   <222> (4)..(4)
   <223> AMIDATION
<400> 55
   Arg Xaa Arg Xaa
   1
<210> 56
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> N(Me)Arg
<220>
   <221> MOD_RES
   <222> (2)..(2)
   <223> Aib
<220>
   <221> MOD_RES
   <222> (3)..(3)
   <223> N(Me)Arg
<220>
   <221> MOD_RES
   <222> (4)..(4)
   <223> alpha.(Me)Phe
<220>
   <221> MOD_RES
   <222> (4)..(4)
   <223> AMIDATION
<400> 56
   Xaa Xaa Xaa Xaa
   1
<210> 57
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> N(Me)Arg
<220>
   <221> MOD_RES
   <222> (2)..(2)
   <223> Aib
<220>
   <221> MOD_RES
   <222> (3)..(3)
   <223> N(Me)Arg
<220>
   <221> MOD_RES
   <222> (4)..(4)
   <223> alpha(Me)Phe
<220>
   <221> MOD_RES
   <222> (4)..(4)
   <223> AMIDATION
<400> 57
   Xaa Xaa Xaa Xaa
   1
<210> 58
   <211> 4
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (2)..(2)
   <223> Aib
<220>
   <221> MOD_RES
   <222> (3)..(3)
   <223> N(Me)Arg
<220>
   <221> MOD_RES
   <222> (4)..(4)
   <223> alpha(Me)Phe
<220>
   <221> MOD_RES
   <222> (4)..(4)
   <223> AMIDATION
<400> 58
   Arg Xaa Xaa Xaa
   1
<210> 59
   <211> 5
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> Aib
<220>
   <221> MOD_RES
   <222> (2)..(2)
   <223> N(Me)Arg
<220>
   <221> MOD_RES
   <222> (3)..(3)
   <223> Aib
<220>
   <221> MOD_RES
   <222> (5)..(5)
   <223> AMIDATION
<400> 59
   Xaa Xaa Xaa Arg Phe
   1               5
<210> 60
   <211> 5
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> Aib
<220>
   <221> MOD_RES
   <222> (2)..(2)
   <223> N(Me)Arg
<220>
   <221> MOD_RES
   <222> (3)..(3)
   <223> Aib
<220>
   <221> MOD_RES
   <222> (4)..(4)
   <223> N(Me)Arg
<220>
   <221> MOD_RES
   <222> (5)..(5)
   <223> AMIDATION
<400> 60
   Xaa Xaa Xaa Xaa Phe
   1               5
<210> 61
   <211> 5
   <212> **PRT**
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> Aib
<220>
   <221> MOD_RES
   <222> (3)..(3)
   <223> Aib
<220>
   <221> MOD_RES
   <222> (4)..(4)
   <223> N(Me)Arg
<220>
   <221> MOD_RES
   <222> (5)..(5)
   <223> AMIDATION
<400> 61
   Xaa Arg Xaa Xaa Phe
   1               5
<210> 62
   <211> 5
   <212> PRT
   <213> Artificial Sequence
<220>
   <223> synthetic peptide
<220>
   <221> MOD_RES
   <222> (1)..(1)
   <223> ACETYLATION
<220>
   <221> MISC_FEATURE
   <222> (1)..(5)
<220>
   <221> MOD_RES
   <222> (1)..(1)
   <223> Aib
<220>
   <221> MOD_RES
   <222> (3)..(3)
   <223> Aib
<220>
   <221> MOD_RES
   <222> (5)..(5)
   <223> alpha(Me)Phe
<220>
   <221> MOD_RES
   <222> (5)..(5)
   <223> AMIDATION
<400> 62
   Xaa Arg Xaa Arg Xaa
   1               5
<210> 63
   <211> 5
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> Aib
<220>
   <221> MOD_RES
   <222> (2)..(2)
   <223> N(Me)Arg
<220>
   <221> MOD_RES
   <222> (3)..(3)
   <223> Aib
<220>
   <221> MOD_RES
   <222> (5)..(5)
   <223> alpha(Me)Phe
<220>
   <221> MOD_RES
   <222> (5)..(5)
   <223> AMIDATION
<400> 63
   Xaa Xaa Xaa Arg Xaa
   1               5
<210> 64
   <211> 5
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> Aib
<220>
   <221> MOD_RES
   <222> (2)..(2)
   <223> N(Me)Arg
<220>
   <221> MOD_RES
   <222> (3)..(3)
   <223> Aib
<220>
   <221> MOD_RES
   <222> (4)..(4)
   <223> N(Me)Arg
<220>
   <221> MOD_RES
   <222> (5)..(5)
   <223> alpha(Me)Phe
<220>
   <221> MOD_RES
   <222> (5)..(5)
   <223> AMIDATION
<400> 64
   Xaa Xaa Xaa Xaa Xaa
   1               5
<210> 65
   <211> 5
   <212> PRT
   <213> Artificial Sequence
<220>
   <223> synthetic peptide
<220>
   <221> MOD_RES
   <222> (1)..(1)
   <223> ACETYLATION
<220>
   <221> MOD_RES
   <222> (1)..(1)
   <223> Aib
<220>
   <221> MOD_RES
   <222> (3)..(3)
   <223> Aib
<220>
   <221> MOD_RES
   <222> (4)..(4)
   <223> N(Me)Arg
<220>
   <221> MOD_RES
   <222> (5)..(5)
   <223> alpha(Me)Phe
<220>
   <221> MOD_RES
   <222> (5)..(5)
   <223> AMIDATION
<400> 65
   Xaa Arg Xaa Xaa Xaa
   1               5

## Description

The present invention relates to combinations of tetra- or pentapeptides having an anti-inflammatory and antiangiogenic activity with insulin for the treatment of diabetes and the associated ocular pathologies. In particular, the combinations according to the invention prevent and counteract symptoms of diabetes such as degenerative processes of the visual apparatus like diabetic retinopathy, cataract, glaucoma and degenerative maculopathy.

### State of the art

Diabetes mellitus (DM) is a metabolic alteration resulting from a decline in insulin activity. In particular, DM may be due to reduced availability of said hormone, an impediment to its normal action, or a combination of those two factors. Two types of DM are known, called type 1 DM and type 2 DM.

In type 1 DM, which affects about 10% of patients with DM, the pancreas fails to produce insulin due to the destruction of the β cells of the islets of Langerhans that produce said hormone, with the result that it must be injected every day, for life. However, the rate of destruction of the β-cells is quite variable, so the onset of the disease may take place rapidly in some individuals, usually children and adolescents, and more slowly in adults.

Type 2 DM, which is much more widespread, affects about 90% of the diabetic population, and has a multifactorial etiology caused by a combination of multiple genetic and environmental factors. The causal factors responsible lead to the onset of the disease by means of two main mechanisms: alteration of insulin secretion and onset of insulin resistance, namely reduced sensitivity of the target tissues to the action of the hormone.

DM is becoming increasingly common; in the USA alone, about 200,000 new cases of diabetes are reported every year. The percentage of the worldwide population suffering from the disease is now estimated by the WHO at 5%, with a prevalence of 25% for woman, and it is estimated that by 2030, over 360 million people worldwide will suffer from DM. The disease increases with age, ranging from 0.5% in those under 30 years old to 10% and over in those aged over 65.

In type 1 DM, wherein there is a total insulin deficiency, and in type 2 DM, which is resistant to dietary treatment and oral antidiabetics, pharmacological treatment involves the administration of insulin as a replacement therapy.

Nowadays, human insulins obtained by amino-acid substitution of porcine insulin or produced from recombinant strains of *Escherichia coli* are mainly used. There are various types of insulin preparations, classified on the basis of the duration of their action: regular or soluble human insulin, insulin lispro, insulin aspart and insulin glulisine, which are fast-acting; NPH human insulin and slow human insulin, which are intermediate-acting; ultra-slow human insulin, which has a long duration of action; and delayed-action insulin analogues such as insulin glargine and insulin detemir.

Insulin is administered by injection into the subcutaneous tissue, preferably of the abdomen. The most common treatment regimen involves three insulin injections to be administered before meals. It is useful to combine said injections with an intermediate-action insulin taken before the evening meal or before going to bed, to cover the overnight requirement.

Other protocols involve a daily injection of an insulin analogue such as glargine or detemir, which covers the basal requirement, and injections of fast-acting insulin at mealtimes. These protocols provide more effective control of the postprandial blood glucose level, but are less effective in maintaining the blood glucose level throughout the day.

The FDA in the USA recently approved a dry form, for respiratory administration using a small inhaler, to improve blood glucose control in patients with type 1 or 2 diabetes. The medicament must be taken before the meal, and is rapidly absorbed.

One characteristic always present in DM is hyperglycaemia which, with time, tends to be associated with major complications of the blood vessels: *macroangiopathy,* a particularly severe, early form of atherosclerosis, which is not specific to diabetes, and *microangiopathy,* an alteration of the circulation in the small arteries, which occurs particularly in the retina, kidneys and nerves, and is specific to diabetes. A particularly disabling long-term complication of DM is diabetic retinopathy (DR), which most often affects diabetics aged between 25 and 60; it is not usually manifested during the first stage of diabetes, but develops exponentially when the patient has suffered from DM for at least ten years. DR is caused by damage to the walls of the blood vessels, especially those of the microcirculation of the visual apparatus; this gives rise to an insufficient supply of blood, and therefore oxygen, to some parts of the retina, which consequently become ischaemic and tend to die. Before this happens, the tissues of the ischaemic areas release growth factors of new blood vessels which, by proliferating in an uncontrolled way, further damage the retinal tissue, compromising its functionality. In its most severe proliferative form, DR is characterised by the presence of intense vascular proliferation (tuft formations), with extremely fragile blood vessels that often tend to break, causing serious retinal damage, which leads to low vision and, in the most severe cases, to blindness. In the less disabling non-proliferative form, DR does not present new blood vessel formation, only microaneurisms; however, they affect not only the small retinal vessels but also larger blood vessels, and sometimes exudates with protein, lipid and carbohydrate deposits, which combine to worsen the eyesight. The non-proliferative form often tends to degenerate to the proliferative form.

A series of studies (Invest Ophthalmol Vis Sci. 1999, 40:3281-3286; Invest Ophthalmol Vis Sci. 2007, 48:5671-5676) demonstrate that the changeover from oral hypoglycaemic treatment to parenteral treatment with insulin, despite better blood glucose control, sometimes paradoxically aggravates DR, because the insulin administered probably stimulates the production of VEGF (vascular endothelial growth factor), a factor that induces abnormal hypervascularization.

In view of these findings, there is great interest in the development of pharmaceutical formulations wherein the hypoglycaemic effect of insulin is combined with active ingredients that counteract the action of VEGF on the degenerative process of retinal vascularization, which underlies DR.

The efficacy of anti-VEGF medicaments in ocular pathologies associated with vascularization disorders in the retina and macula (aflibercept, ranibizumab, bevacizumab, pegaptinib, lampalizumab and abicipar pegol) is closely associated with the form of administration, which must necessarily be intravitreal injection, with almost monthly administrations.

Small peptides are also known as VEGF inhibitors (Mol Cancer Ther, 13, (2014) 1092-1104; WO2008017372), and have proved effective in animal models of retinal hypervascularization (Investigative Ophthalmology & Visual Science, June 2003, Vol. 44, No. 6; Investigative Ophthalmology and Visual Science, 56, (2015) 2392-2406). Said treatments are highly incapacitating, expensive, do not allow effective concentrations of the active ingredient to be maintained for a long time, and are often ineffective in the most severe cases of full-blown DR. No more convenient and effective forms of administration for the prevention and treatment of DR are currently known.

Accordingly, there is a need to develop innovative treatment strategies that combat these ocular pathologies, which often lead to blindness, in a less traumatic and more effective way.

The pharmaceutical formulations combined with insulin treatment described below allow the prevention and treatment of ocular pathologies associated with diabetes, using non-traumatic administration routes which, at limited costs, allow continuous treatments and effective, constant levels of the active ingredient over time.

### Description of the invention

The invention relates to peptides of general formula (I):

L₁-X₁-X₂-X₃-X₄

wherein:
L₁ is H, or acyl, or an optionally N-acylated and/or N-alkylated and/or Cα-alkylated amino acid selected from Glu, Gln, Pro, hydroxy-Pro, Azt, Pip, pGlu, Aib, Ac4c, Ac5c and Ac6c;
X₁ and X₃, which can be the same or different, are an optionally N-alkylated and/or Cα-alkylated basic amino acid, selected from Arg, Orn and optionally guanidylated Lys, and phenylalanine substituted at the *meta* or *para* positions with an amino or guanidino group;
X₂ is an optionally N-alkylated amino acid selected from Glu, Lys, α-methyl-leucine, α-methyl-valine, α-methyl-glutamic acid, Aib, Ac4c, Ac5c and Ac6c;
X₄ is a hydrophobic amino acid which is amidated or non-amidated at the C-terminal end and optionally Cα-alkylated, selected from Phe, h-Phe, Tyr, Trp, 1-Nal, 2-Nal, h-1-Nal, h-2-Nal, Cha, Chg, Phg
and the salts thereof for therapeutic use in combination with an insulin for the treatment of ocular pathologies associated with diabetes.

The peptides of formula I are known from WO 08017372.

The following are the conventional abbreviations used for some of the unnatural amino acids which can be included in the formulas of the peptides according to the invention:
Azt = azetidine acid, Pip = pipecolic acid, Aib = α-amino-isobutyric acid, Ac4c = 1-aminocyclobutane-1-carboxylic acid, Ac5c = 1-aminocyclopentane-1-carboxylic acid, Ac6c = 1-aminocyclohexane-1-carboxylic acid, h-Phe = homophenylalanine, 1-Nal = β-1-naphthyl-alanine, 2-Nal = β-2-naphthyl-alanine, h-1-Nal = homo-β-1-naphthyl-alanine, h-2-Nal = homo-β-2-naphthyl-alanine, Cha = cyclohexyl-alanine, Chg = cyclohexyl-glycine, Phg = phenyl-glycine, pGlu = pyroglutamic acid.

The preferred peptides for use according to the invention are:
Ac-Arg-Glu-Arg-Phe-NH₂; pGlu-Arg-Glu-Arg-Tyr-OH; Glu-Arg-Glu-Arg-Phe-NH₂; Ac-Arg-Glu-Arg-Tyr-NH₂; Ac-Arg-Glu-Arg-Trp-NH₂; Ac-Arg-Glu-N(Me)Arg-Phe-NH₂; Ac-Arg-Glu-N(Me)Arg-Tyr-NH₂; Ac-Arg-Glu-N(Me)Arg-Trp-NH₂; pGlu-Arg-Glu-N(Me)Arg-Phe-NH₂; pGlu-Arg-Glu-N(Me)Arg-Tyr-NH₂; pGlu-Arg-Glu-N(Me)Arg-Trp-NH₂; pGlu-Arg-Glu-Arg-Phe-NH₂; pGlu-Arg-Glu-Arg-Tyr-NH₂; pGlu-Arg-Glu-Arg-Trp-NH₂; Ac-Arg-Aib-Arg-Phe-NH₂; Ac-Arg-Aib-Arg-Tyr-NH₂; Ac-Arg-Aib-Arg-Trp-NH₂; Ac-Aib-Arg-Aib-Arg-Phe-NH₂; Ac-Arg-Aib-N(Me)Arg-Phe-NH₂; Ac-Arg-Aib-N(Me)Arg-Tyr-NH₂; Ac-Arg-Aib-N(Me)Arg-Trp-NH₂; pGlu-Arg-Aib-N(Me)Arg-Phe-NH2; Glu-Arg-Aib-N(Me)Arg-Tyr-NH₂; pGlu-Arg-Aib-N(Me)Arg-Trp-NH₂; pGlu-Arg-Aib-Arg-Phe-NH₂; pGlu-Arg-Aib-Arg-Tyr-NH₂; pGlu-Arg-Aib-Arg-Trp-NH₂; Ac-Arg-Ac5c-Arg-Phe-NH₂; Ac-Arg-Ac5c-Arg-Tyr-NH₂; Ac-Arg-Ac5c-Arg-Trp-NH₂; Ac-Arg-Ac5c-N(Me)Arg-Phe-NH₂; Ac-Arg-Ac5c-N(Me)Arg-Tyr-NH₂; Ac-Arg-Ac5c-N(Me)Arg-Trp-NH₂; pGlu-Arg-Ac5c-N(Me)Arg-Phe-NH₂; pGlu-Arg-Ac5c-N(Me)Arg-Tyr-NH₂; pGlu-Arg-Ac5c-N(Me)Arg-Trp-NH₂; pGlu-Arg-Ac5c-Arg-Phe-NH₂; pGlu-Arg-Ac5c-Arg-Tyr-NH₂; pGlu-Arg-Ac5c-Arg-Trp-NH₂; Ac-Arg-Glu-Arg-Phe-OH; Ac-Arg-Glu-Arg-Tyr-OH; Ac-Arg-Glu-Arg-Trp-OH; Ac-Arg-Glu-N(Me)Arg-Tyr-OH; pGlu-Arg-Glu-N(Me)Arg-Phe-OH; pGlu-Arg-Glu-Arg-Trp-OH; Ac-Arg-Aib-Arg-Phe-OH; Ac-Arg-Aib-N(Me)Arg-Phe-OH; pGlu-Arg-Aib-N(Me)Arg-Tyr-OH; pGlu-Arg-Aib-Arg-Trp-OH; Ac-Arg-Ac5c-Arg-Phe-OH; Ac-Arg-Ac5c-N(Me)Arg-Tyr-OH; pGlu-Arg-Ac5c-N(Me)Arg-Trp-OH;pGlu-Arg-Ac5c-Arg-Trp-OH; Ac-N(Me)Arg-Aib-Arg-Phe-NH₂; Ac-N(Me)Arg-Aib-N(Me)Arg-Phe-NH₂; Ac-Arg-Aib-N(Me)Arg-Phe-NH₂; Ac-Arg-Aib-Arg-α(Me)Phe-NH₂; Ac-N(Me)Arg-Aib-Arg-α(Me)Phe-NH₂; Ac-N(Me)Arg-Aib-N(Me)Arg- α(Me)Phe-NH₂; Ac-Arg-Aib-N(Me)Arg-α(Me)Phe-NH₂; Ac-Aib-N(Me)Arg-Aib-Arg-Phe-NH₂; Ac-Aib-N(Me)Arg-Aib-N(Me)Arg-Phe-NH₂; Ac-Aib-Arg-Aib-N(Me)Arg-Phe-NH₂; Ac-Aib-Arg-Aib-Arg-α(Me)Phe-NH₂; Ac-Aib-N(Me)Arg-Aib-Arg-α(Me)Phe-NH₂; Ac-Aib-N(Me)Arg-Aib-N(Me)Arg-α(Me)Phe-NH₂; Ac-Aib-Arg-Aib-N(Me)Arg-α(Me)Phe-NH₂.

The peptides Ac-Arg-Aib-Arg-α(Me)Phe-NH₂ and Ac-Aib-Arg-Aib-Arg-α(Me)Phe-NH₂ are particularly preferred.

Systemic injective administration of the peptides according to the invention, in particular subcutaneously and combined with insulin treatment, counteracts the onset of the ocular pathologies associated with diabetes and leads to their regression when already manifested, without exhibiting any toxic effects.

This is surprising, because the difficulty encountered by systemically administered medicaments in general, and peptides in particular, in crossing the blood-vitreous barrier at pharmacologically effective concentrations is well known, as in the case of aflibercept, ranibizumab, bevacizumab, pegaptinib, lampalizumab and abicipar pegol. Said medicaments are not only ineffective against ocular pathologies, but also give rise to serious adverse effects.

In another aspect thereof, the invention provides pharmaceutical formulations comprising a combination of a peptide, as defined above, with insulin, in a form suitable for separate, simultaneous or sequential administration.

These formulations can contain peptides and insulin in the same dosage form or can be in kit form, consisting of separate dosage units, such as ampoules for injection containing solutions of peptides of formula I and syringes pre-filled with insulin for subcutaneous administration.

For the recommended therapeutic uses in combination with insulin, the peptides according to the invention can be formulated as such, or in the form of salts, in liquid or solid pharmaceutical compositions, which can be administered by all the routes conventionally used for insulin treatment, such as subcutaneously, intramuscularly, intravenously, orally, nasally, sublingually, topically, transdermally or by inhalation. Subcutaneous administration is preferred, this route normally being used for insulin. The unit doses of the peptide in humans can vary within wide ranges, typically from 100 µg to 500 mg per dose, and preferably between 1 mg and 200 mg. Said doses can easily be determined by the expert, depending on the stage of the disease and taking account of the patient's weight, gender and age.

Suitable forms of insulin include regular or soluble human insulin, insulin lispro, insulin aspart, insulin glulisine, NPH human insulin, slow human insulin, ultra-slow human insulin, which has a long duration of action; and delayed-action insulin analogues such as insulin glargine and insulin detemir.

The following examples illustrate the invention in greater detail.

EXAMPLE 1 - Formulations containing Ac-Arg-Aib-Arg-α(Me)Phe-NH₂ succinate or Ac-Aib-Arg-Aib-Arg-α(Me)Phe-NH₂ succinate and insulin for subcutaneous administration.

Preparation of 1 L of 80 mg/mL solution of Ac-Arg-Aib-Arg-α(Me)Phe-NH₂ succinate, pH 7.1-7.3, 290-350 mOsmol/Kg (solution 2):
80 g of Ac-Arg-Aib-Arg-α(Me)Phe-NH₂ succinate and 2.0 g of NaH₂PO₄•H₂O are dissolved in 800 mL of pyrogen-free water, the pH is corrected to 7.1-7.3 with IN NaOH, and the solution is made up to a volume of 1 L with pyrogen-free water and sterilized by filtration through 0.22 µm.

Preparation of 1 L of 91 mg/mL solution of Ac-Aib-Arg-Aib-Arg-α(Me)Phe-NH₂ succinate, pH 7.1-7.3, 290-350 mOsmol/Kg (solution 4):
91 g of Ac-Aib-Arg-Aib-Arg-α(Me)Phe-NH₂ succinate and 2.0 g of NaH₂PO₄•H₂O are dissolved in 800 mL of pyrogen-free water, the pH is corrected to 7.1-7.3 with IN NaOH, and the solution is made up to a volume of 1 L with pyrogen-free water and sterilized by filtration through 0.22 µm.

Table 1 shows the insulin/peptide formulations prepared by mixing 1 mL of 20 U/mL insulin (solution 1) with 1 mL of the peptide solutions reported above (solutions 2 and 4).

**Table 1 - The insulin/peptide formulations are prepared by mixing 1 mL of 20 U/mL insulin with 1 mL of the 80 mg/mL solution of peptide Ac-Arg-Aib-Arg-α(Me)Phe-NH₂ or the 91 mg/mL solution of peptide Ac-Aib-Arg-Aib-Arg-α(Me)Phe-NH₂).**

| Solution | Insulin (U*) | Peptide (mg/mL) | |
|---|---|---|---|
| 3 | 20 | Ac-Arg-Aib-Arg-α(Me)Phe-NH₂ | 80 |
| 5 | 20 | Ac-Aib-Arg-Aib-Arg-α(Me)Phe-NH₂ | 91 |

| | | | |
|---|---|---|---|
| *the commercial formulation (40 U/mL) is diluted 1:1 with saline solution | | | |

EXAMPLE 2 - Efficacy in the treatment of diabetes-prone BioBreeding rats.

The efficacy of formulation 3, described in example 1, in the treatment of the retinal vascular alterations associated with diabetes, was evaluated using as experimental model DP BB rats (Diabetes-Prone BioBreeding rats, M&B Bomholtvej, Denmark), analyzing the retinal function of the treated animals by electroretinography (ERG) and the state of vascularization of the retina by quantitative immunohistochemical techniques.

DP BB rats are a useful experimental model of type 1 diabetes, because at the age of 12-13 weeks they spontaneously develop a type of diabetes which, in various respects, closely resembles that observed in human beings, involving the onset of degenerative processes of the eyesight due to the formation of abnormal vascularization of the retina.

60 3-week old DP BB rats of both sexes (30 male and 30 female) were used. The experiments were conducted in accordance with the ARVO Statement for the Use of Animals in Ophthalmic and Visual Research and in compliance with EEC Directive 86/609. The animals were housed in a controlled environment at 23±1°C, with 50±5% humidity, a 12h light/dark cycle, and unlimited access to food and water. The animals were observed from the 3rd to 30th weeks of life, monitoring various parameters, including weight, blood glucose, urine glucose and blood insulin. In particular, the onset of diabetes was evaluated by measuring blood glucose with the One-Touch profile (LifeScan, Milpitas, CA) twice a week, and every day from the 13th week; urine glucose with Clinistix (Bayer Diagnostica, Basingstoke, United Kingdom), twice a week; insulin levels twice a week, with an RIA Kit (BioRad, Milan, Italy). The rats were considered hyperglycaemic when their blood glucose level was greater than 12 mM but less than 15 mM, and diabetic when their blood glucose levels exceeded 15 mM in two successive determinations. The young (3-week-old) animals had blood glucose levels ranging between 4 and 5 mM; at 10 weeks the blood glucose values ranged between 6 and 8 mM; at 15 weeks the animals were clearly diabetic, with blood glucose values exceeding 15 mM and weights ranging between 200 and 270 g. For this reason, from the 15th week the rats were given insulin treatment, administered subcutaneously, with two daily doses ranging between 1 and 2U (Humulin R, Eli Lilly France SA, Paris, France), depending on the blood glucose level.

The solutions used for the treatments are as described in Example 1.

The study design involved 6 groups of 10 rats, 5 male and 5 female: Group A: animals euthanized in the 4th week, which presented blood glucose levels of 4-5 mM and absence of full-blown diabetes, and did not receive any treatment; Group B: animals euthanized in the 15th week, which reached blood glucose levels close to 15 mM, not having received any treatment, and were considered diabetic; Group C: animals which received 2U of insulin subcutaneously twice a day from the 15th to the 30th weeks; Group D: animals which received 2U of insulin+4 mg of Ac-Arg-Aib-Arg-α(Me)Phe-NH₂ subcutaneously twice a day from the 15th to the 30th week; Group E: animals which received 4 mg of Ac-Arg-Aib-Arg-α(Me)Phe-NH₂ subcutaneously twice a day from the 3rd to the 15th week; Group F: animals which received 4 mg of Ac-Arg-Aib-Arg-α(Me)Phe-NH₂ subcutaneously twice a day from the 3rd to the 15th week and 2U of insulin+4 mg of Ac-Arg-Aib-Arg-α(Me)Phe-NH₂ subcutaneously twice a day from the 15th to the 30th week. The retinal functionality of the animals in each group was evaluated by ERG before euthanasia and removal of the retinas for quantitative histochemical analysis.

### Assessment of retinal function

The efficacy of formulation 1 of example 1 in preventing the onset of DM or obtaining its regression was evaluated by analyzing the retinal function of the animals treated under the various conditions. Retinal functionality was evaluated by ERG, using a scotopic full-field ERG. The electrophysiological signals were recorded as reported in J. Neurochem. 2011; 119:1317-1329. The rats were adapted to darkness overnight and anaesthetized by intraperitoneal injection of avertin. The pupils were dilated with 0.5% w/w of atropine. The responses (cones and rods together) were stimulated by flashes of light of different intensities, ranging from 3.4 to 1 log cd-s.m², produced with a Ganzfeld stimulator (Biomedica Mangoni, Pisa, Italy). The wave amplitude was measured at a fixed time of 8 ms from the stimulus, to minimise non-photoreceptor contamination. The amplitude of wave b was measured from the trough of the wave to the peak of wave b. Of the five potential oscillators (PO), only PO2, PO3 and PO4, generated by flashes of light at 1 log cd-s.m², were taken into consideration. For each PO, the trough-peak amplitude was measured and the amplitudes were totalled (SOPs).

As shown in Table 2, daily subcutaneous administrations of Ac-Arg-Aib-Arg-α(Me)Phe-NH₂ prevent the formation of retinal vascular damage at the onset stage of diabetes (group E), and when the disease is full-blown and requires insulin treatment of the animals (group F). However, insulin treatment alone causes major retinal vascular damage, seriously compromising the eyesight (Group C), whereas treatment with Ac-Arg-Aib-Arg-α(Me)Phe-NH₂ + insulin causes regression of the retinal damage and good recovery of the eyesight (group D).

**Table 2 - Evaluation of retinal function. The retinal function was evaluated by ERG, using a scotopic full-field ERG. The rats, adapted to darkness overnight, were anaesthetized by intraperitoneal injection of avertin, and the pupils were dilated with 0.5% w/w of atropine. The responses (cones and rods together) were stimulated by flashes of light (1 log cd-s.m², produced with a Ganzfeld stimulator (Biomedica Mangoni, Pisa, Italia). The wave amplitude was measured at a fixed time of 8 ms after the stimulus, to minimise non-photoreceptor contamination. The amplitude of wave b was measured from the trough of the wave to the peak of wave b. Of the five potential oscillators (PO), only PO2, PO3 and PO4, generated by flashes of light at 1 log cd-s.m², were taken into consideration. For each PO, the trough-peak amplitude was measured and the amplitudes were totalled (SOPs). Each group was formed by 10 DP BB rats, 5 male and 5 female. From the 15th week the animals spontaneously developed full-blown diabetes accompanied by degenerative processes of the eyesight caused by an alteration of the retinal vascular situation. Group A: animals euthanized in the 4th week, which presented blood glucose levels of 4-5 mM, absence of full-blown diabetes, and did not receive any treatment. Group B: animals euthanized in the 15th week, which reached blood glucose levels close to 15 mM, not having received any treatment, and were considered diabetic. Group C: animals which received 2U of insulin (50 µL sol. 1) subcutaneously twice a day from the 15th to 30th weeks. Group D: animals which received 2U of insulin+4 mg of Ac-Arg-Aib-Arg-α(Me)Phe-NH₂ (100 µL sol. 3) subcutaneously twice a day from the 15th to 30th weeks; Group E: animals which received 4 mg of Ac-Arg-Aib-Arg-α(Me)Phe-NH₂ (50 µL sol. 2) subcutaneously twice a day from the 3rd to 15th weeks; Group F: animals which received 4 mg of Ac-Arg-Aib-Arg-α(Me)Phe-NH₂ (50 µL sol. 2) subcutaneously twice a day from the 3rd to 15th weeks and 2U of insulin+4 mg of Ac-Arg-Aib-Arg-α(Me)Phe-NH₂ (100 µL sol. 3) subcutaneously twice a day from the 15th to 30th weeks.**

| Group | Treatment (b.i.d) | | Euthanasia | SOPs |
|---|---|---|---|---|
| | (µL)* | (week) | | (µV) |
| A | none | 3rd | 3rd | 250± 11 |
| B | none | 15th | 15th | 180±14 |
| C | 50 sol.1 | 15th-30th | 30th | 120±12 |
| D | 100 sol.3 | 15th-30th | 30th | 220±15 |
| E | 50 sol.2 | 3rd-15th | 15th | 230±18 |
| F | 50 sol.2 | 3rd-15th | 30th | 245±13 |
| | 100 sol.3 | 15th-30th | | |

| | | | | |
|---|---|---|---|---|
| *solutions as described in example 1 | | | | |

**Immunohistochemical tests and quantitative analysis of state of retinal vascularization** - The state of vascularization of the retina of the treated animals was studied on the whole retinas, using antibodies directed against CD31 (BD Biosciences, San Diego, CA, USA), an important marker for the endothelial cells. The immunohistochemical tests on the retinas and quantitative analysis of the neovascular tuft formations, vascular and avascular areas, were conducted as reported in the literature (Exp Eye Res. 2013; 111:27-35; Invest Ophthalmol Vis Sci. 2012; 53:2181-2192; J Neurochem. 2011; 119:1317-1329; Invest Ophthalmol Vis Sci. 2006; 47:2125-2134). After euthanasia of the animals and enucleation of the eyes, the retinas were removed, fixed by immersion for 1.5h in 4% paraformaldehyde in 0.1M phosphate buffer at 48C°, transferred to 25% saccharose in 0.1M phosphate buffer PB, and stored at 48°C.

Subsequently, when all the samples had been collected, the retinas thus treated were thawed and incubated for 72h at 48°C with the CD31 antibody (1:50 in 0.1M phosphate buffer containing 0.5% Triton X-100). The assembled whole was then incubated for 48h at 48°C with the AlexaFluor 488 secondary antibody (Molecular Probes, Eugene, OR, USA; 1:200 in 0.1M phosphate buffer). Finally, the retinas thus treated were washed in 0.1M phosphate buffer, mounted on microscopy slides coated with gelatin and covered with a coverslip, using an 0.1M glycerin phosphate buffer mixture.

The retinal vascularization images were acquired with an epifluorescence microscope (Eclipse E800; Nikon Corp., Amsterdam, Netherlands) using a digital camera converter (SD-Filc camera; Nikon Corp.). The electronic images were processed with image-processing software (Adobe Photoshop; Adobe Systems, Inc., Mountain View, CA, USA) to allow quantitative analysis of the results. For each experimental condition, the quantitative data originated from 20 retinas of 10 different rats. Table 3 shows the mean data of the samples analysed.

**Table 3 - Immunohistochemical tests on retinas and quantitative analysis of neovascular tuft formations. The state of vascularization of the retinas of the treated animals was studied on the whole retinas, using antibodies directed against CD31 (BD Biosciences, San Diego, CA, USA), an important marker for the endothelial cells. After euthanasia of the animals and enucleation of the eyes, the retinas were removed, fixed by immersion for 1.5h in 4% paraformaldehyde in 0.1M phosphate buffer at 48C°, transferred to 25% saccharose in 0.1M phosphate buffer PB, and stored at 48°C. Subsequently, when all the samples had been collected, the retinas thus treated were thawed and incubated for 72h at 48°C with the CD31 antibody (1:50 in 0.1M phosphate buffer containing 0.5% Triton X-100). The assembled whole was then incubated for 48h at 48°C with the AlexaFluor 488 secondary antibody (Molecular Probes, Eugene, OR, USA; 1: 200 in 0.1M phosphate buffer). Finally, the retinas thus treated were washed in 0.1M phosphate buffer, mounted on microscopy slides coated with gelatin and covered with a coverslip, using an 0.1M glycerin phosphate buffer mixture. The retinal vascularization images were acquired with an epifluorescence microscope (Eclipse E800; Nikon Corp., Amsterdam, Netherlands) using a digital camera converter (SD-Fi1c camera; Nikon Corp.). The electronic images were processed with image-processing software (Adobe Photoshop; Adobe Systems, Inc., Mountain View, CA, USA) to allow quantitative analysis of the results. For each experimental condition, the quantitative data originated from 20 retinas of 10 different rats. The study design involved 6 groups of 10 DP BB rats, 5 male and 5 female. From the 15th week the animals spontaneously developed full-blown diabetes accompanied by degenerative processes of the eyesight caused by an alteration of the retinal vascular situation with neovascularization tuft formations. The areas of neovascularization are expressed as the ratio between the area of neovascularization present in the animals which are not yet diabetic (Group A ≈ 0.1% of the retinal area) and those in the group. Group A: animals euthanized in the 4th week, which presented blood glucose levels of 4-5 mM, absence of full-blown diabetes, and did not receive any treatment. Group B: animals euthanized in the 15th week, which reached blood glucose levels close to 15 mM, not having received any treatment, and were considered diabetic. Group C: animals which received 2U of insulin subcutaneously twice a day from the 15th to the 30th weeks (50 µL sol. 1). Group D: animals which received 2U of insulin + 4 mg of Ac-Arg-Aib-Arg-α(Me)Phe-NH₂ (100 µL sol. 3) subcutaneously twice a day from the 15th to 30th weeks) Group E: animals which received 4 mg of Ac-Arg-Aib-Arg-α(Me)Phe-NH₂ (50 µL sol. 2) subcutaneously twice a day from the 3rd to 15th weeks; Group F: animals which received 4 mg of Ac-Arg-Aib-Arg-α(Me)Phe-NH₂ (50 µL sol. 2) subcutaneously twice a day from the 3rd to 15th weeks and 2U of insulin+4 mg of Ac-Arg-Aib-Arg-α(Me)Phe-NH₂ (100 µL sol. 3) subcutaneously twice a day from the 15th to 30th weeks.**

| Group | Treatment (b.i.d) | | Euthanasia | NTA° of group/NTA of group A* |
|---|---|---|---|---|
| | (µL)* | (week) | | |
| A | None | 3rd | 3rd | 1.00±0.11 |
| B | None | 15th | 15th | 7.10±0.18 |
| C | 50 sol.1 | 15th-30th | 30th | 15.02±0.62 |
| D | 100 sol.3 | 15th-30th | 30th | 2.12±0.12 |
| E | 50 sol.2 | 3rd-15th | 15th | 1.51±0.12 |
| F | 50 sol.2 | 3rd-15th | 30th | 1.10±0.11 |
| | 100 sol.3 | 15th-30th | | |

| | | | | |
|---|---|---|---|---|
| *solutions as described in example 1 °NTA Neovascularization Tuft Area *Animals not yet diabetic | | | | |

As shown in Table 3, daily subcutaneous administrations of Ac-Arg-Aib-Arg-α(Me)Phe-NH₂ prevent the formation of retinal vascular damage at the onset stage of diabetes (group E), and when the disease is full-blown and requires insulin treatment of the animals (group F). However, insulin treatment alone causes a major increase in NTA indicating retinal vascular damage, which seriously compromises the eyesight, as confirmed by the data in Table 2 (group C), whereas treatment with Ac-Arg-Aib-Arg-α(Me)Phe-NH₂ + insulin causes regression of the retinal damage and good recovery of the eyesight (group D, Table 2).

EXAMPLE 3 - Efficacy of formulation 5 described in example 1 in the treatment of rats rendered diabetic by treatment with streptozotocin.

The efficacy of formulation 5, described in example 1, in the treatment of retinal vascular alterations associated with diabetes, was evaluated, using as experimental model of type 1 diabetes 8-week-old Sprague-Dawley (SD) rats of both sexes (170-250 g), rendered diabetic with a single intraperitoneal injection of 150 mg/Kg of streptozotocin (STZ; Sigma, St. Louis, MO, USA; Eur J Sci Res ISSN 1450-216X Vol.32 No.3, 2009, pp.398-402), called SD-STZ rats. The severity of the diabetic state was evaluated by daily monitoring of body weight, clinical signs and blood glucose levels.

The retinal vascular alterations manifested in the diabetic animals were evaluated, as reported in example 2, by electroretinography (ERG) for the retinal function and by quantitative immunohistochemical techniques to determine the state of vascularization of the retina.

The dose of STZ was prepared at the time of use by dissolving the compound in 0.05M citrate buffer, pH 4.5. For the STZ administration, the animals were placed in the dorsal position and the injection site was disinfected and buffered with an iodine-povidone solution; the i.p. injection was performed in the caudal abdominal cavity of the rats, using a sterile 25 g needle. The severity of the diabetes caused by administration of STZ was monitored daily, evaluating blood glucose levels with the One-Touch profile (LifeScan, Milpitas, CA), every day; urine glucose with Clinistix (Bayer Diagnostica, Basingstoke, United Kingdom), twice a week; insulin levels, twice a week, with an RIA Kit (BioRad, Milan, Italy). The animals were considered diabetic if the blood glucose was higher than 6-8 mM 24 h after treatment with STZ and exceeded 15 mM 2-3 days after treatment with STZ, when the blood glucose level the animals were treated subcutaneously twice a day with insulin at doses ranging between 1 and 2U (Humulin R, Eli Lilly France SA, Paris, France), depending on the blood glucose level.

50 8-week old SD rats of both sexes (30 male and 30 female) were used. The animals were housed in a controlled environment at 23±1°C, with 50±5% humidity, a 12h light/dark cycle, and unlimited access to food and water.

The solutions used for the treatments were as described in Example 1.

The study design involved 5 groups of 10 rats, 5 male and 5 female: Group A: SD rats, euthanized immediately before i.p. administration of STZ, which presented no signs of diabetes, had blood glucose levels of 4-5 mM, and received no treatment; Group B: SD-STZ5w rats which, 24 h after administration of STZ, already exhibited blood glucose levels >12 mM, which received no treatment and were euthanized 5 weeks after treatment with STZ; Group C: SD-STZ5w rats which, 24 h after treatment with STZ and for the subsequent 5 weeks, received 4.55 mg of Ac-Aib-Arg-Aib-Arg-α(Me)Phe-NH₂ (100 µL sol. 5) subcutaneously, twice a day, and were euthanized 5 weeks after treatment with STZ; Group D: SD-STZ10w animals which, 24h after treatment with STZ and for the subsequent 10 weeks, received 2U of insulin (50 µL sol. 1) subcutaneously twice a day; Group E: SD-STZ10w animals which, 24h after treatment with STZ and for the subsequent 10 weeks received 2U of insulin + 4.55 mg of Ac-Aib-Arg-Aib-Arg-α(Me)Phe-NH₂ (100 µL sol. 5) subcutaneously twice a day. The retinal functionality of the animals in each group was evaluated by ERG before euthanasia and removal of the retinas for quantitative histochemical analysis.

**Evaluation of retinal function** - The efficacy of formulation 5, described in example 1, in preventing the onset of DM or obtaining its regression, was evaluated by analyzing the retinal function of the animals treated under the various conditions, as described in detail in example 2.

As shown in Table 4, daily administrations of Ac-Aib-Arg-Aib-Arg-α(Me)Phe-NH₂ prevent *ab initio* the formation of retinal vascular damage at the stage of onset and development of diabetes (groups C and E). However, insulin treatment alone gives rise to major retinal vascular damage, seriously compromising the eyesight (groups B and D).

**Table 4 - Assessment of retinal function. Retinal function was evaluated by ERG, as reported in detail in example 2.**

| Group | Treatment (b.i.d) | | Euthanasia° | Animals | SOPs |
|---|---|---|---|---|---|
| | (µL)* | (h, hours; w, weeks°) | | | (µV) |
| A | None | - | 0 | SD | 250±13 |
| B | None | 0 | 5w | SD-STZ5w | 100±16 |
| C | 100 sol. 5 | 24h-5w | 5w | SD-STZ5w | 240±15 |
| D | 50 sol. 1 | 24h-10w | 10w | SD-STZ10w | 80±10 |
| E | 100 sol. 5 | 24H-10w | 10w | SD-STZ10w | 245±13 |

| | | | | | |
|---|---|---|---|---|---|
| °Time of euthanasia after treatment with STZ; h hours; w weeks *Solutions as described in example 1 | | | | | |

The study design involved 5 groups of 10 rats, 5 male and 5 female. Each group was formed by 10 SD-STZ rats, 5 male and 5 female, the animals from 8-week-old Sprague-Dawley (SD) rats of both sexes (170-250 g), rendered diabetic with a single intraperitoneal injection of 150 mg/Kg of streptozotocin (STZ; Sigma, St. Louis, MO, USA; Eur J Sci Res ISSN 1450-216X Vol.32 No 3, 2009, pp.398-402), called SD-STZ rats, which presented full-blown diabetes only 24h after treatment with STZ.

Group A: SD rats, euthanized immediately before i.p. administration of STZ, which presented no signs of diabetes, had blood glucose levels of 4-5 mM, and received no treatment; Group B: SD-STZ5w rats which, 5 weeks after administration of STZ, exhibited full-blown diabetes, with very high blood glucose levels, received no treatment, and were euthanized 5 weeks after treatment with STZ; Group C: SD-STZ5w rats which, 24h after treatment with STZ and until the 5th week, received 2U of insulin + 4.55 mg of Ac-Aib-Arg-Aib-Arg-α(Me)Phe-NH₂ (100 µL sol. 5) subcutaneously, twice a day, and were euthanized 5 weeks after treatment with STZ; Group D: SD-STZ10w animals which, 24h after treatment with STZ and until the 10th week, received 2U of insulin (50 µL sol. 1) subcutaneously twice a day; Group E: SD-STZ10w animals which, 24h after treatment with STZ and until the 10th week, received 2U of insulin + 4.55 mg of Ac-Aib-Arg-Aib-Arg-α(Me)Phe-NH₂ (100 µL sol. 5) subcutaneously twice a day.

**Immunohistochemical tests and quantitative analysis of state of retinal vascularization.** The efficacy of formulation 5, described in example 1, in preventing the onset of DM or obtaining its regression, was evaluated quantitatively by histochemical techniques as described in detail in example 2.

For each experimental condition, the quantitative data originated from 20 retinas of 10 different rats. Table 5 shows the mean data of the samples analysed.

**Table 5 - Immunohistochemical tests on retinas and quantitative analysis of neovascular tuft formations. The state of vascularization of the retina of the treated animals was studied as reported in example 2. For each experimental condition, the quantitative data originated from 20 retinas of 10 different rats. The study design involved 5 groups of 10 rats, 5 male and 5 female, rendered diabetic by treatment with STZ. Only 24h after treatment the animals presented full-blown diabetes, eventually accompanied by degenerative processes of the eyesight caused by an alteration of the retinal vascular situation with neovascularization tuft formations. The areas of neovascularization are expressed as the ratio between the area of neovascularization present in the animals which are not yet diabetic (Group A ≈ 0.1% of the retinal area) and that of the group. Group A: SD rats, euthanized immediately before i.p. administration of STZ, which presented no signs of diabetes, had blood glucose levels of 4-5 mM, and received no treatment; Group B: SD-STZ5w rats which, 5 weeks after administration of STZ, exhibited full-blown diabetes, with very high blood glucose levels, received no treatment, and were euthanized 5 weeks after treatment with STZ; Group C: SD-STZ5w rats which, from 24 h after treatment with STZ until the 5th week, received 2U of insulin + 4.55 mg of Ac-Aib-Arg-Aib-Arg-α(Me)Phe-NH₂ (100 µL sol. 5) subcutaneously, twice a day, and were euthanized 5 weeks after treatment with STZ; Group D: SD-STZ10w animals which, from 24h after treatment with STZ until the 10th week, received 2U of insulin (50 µL sol. 1) subcutaneously twice a day; Group E: SD-STZ10w animals which, from 24h after treatment with STZ until the 10th week received 2U of insulin + 4.55 mg of Ac-Aib-Arg-Aib-Arg-α(Me)Phe-NH₂ (100 µL sol. 5) subcutaneously twice a day.**

| Group | Treatment (b.i.d) | | Euthanasia°° | Animals | NTA° group/NTA group A* |
|---|---|---|---|---|---|
| | (µL)** | (h, hours; w, weeks°°) | | | |
| A | None | - | 0 | SD | 1.00±0.12 |
| B | None | 0 | 5w | SD-STZ5w | 13.81±0.29 |
| C | 100 sol. 5 | 24h-5w | 5w | SD-STZ24h | 1.09±0.13 |
| D | 50 sol. 1 | 24h-10w | 10w | SD-STZ10w | 17.11±0.44 |
| E | 100 sol. 5 | 24h-10w | 10w | SD-STZ10w | 1.48±0.11 |

| | | | | | |
|---|---|---|---|---|---|
| °NTA Neovascularization Tuft Area *Animals not yet diabetic °°Time of euthanasia after treatment with STZ; h hours; w weeks **Solutions as described in example 1 | | | | | |

As shown in Table 5, daily administrations of Ac-Aib-Arg-Aib-Arg-α(Me)Phe-NH₂ prevent the formation of retinal vascular damage at the stage of onset of diabetes (groups C and E). However, the absence of treatment (group B) or insulin treatment alone (group D) leads to a major increase in NTA, indicating retinal vascular damage, which seriously compromises the eyesight, as confirmed by the data in Table 3 (group D).

## Claims

1. Peptides of general formula (I):
L₁-X₁-X₂-X₃-X₄,
wherein:
L₁ is H, or acyl, or an optionally N-acylated and/or N-alkylated and/or Cα-alkylated amino acid selected from Glu, Gln, Pro, hydroxy-Pro, Azt, Pip, pGlu, Aib, Ac4c, Ac5c, Ac6c;
X₁ and X₃, which can be the same or different, are an optionally N-alkylated and/or Cα-alkylated basic amino acid, selected from Arg, Orn and optionally guanidylated Lys, and phenylalanines substituted at the *meta* or *para* positions with an amino or guanidino group;
X₂ is an optionally N-alkylated amino acid, selected from Glu, Lys, α-methyl-leucine, α-methyl-valine, α-methyl-glutamic acid, Aib, Ac4c, Ac5c, Ac6c;
X₄ is a hydrophobic amino acid which is amidated or non-amidated at the C-terminal end and optionally Cα-alkylated, selected from Phe, h-Phe, Tyr, Trp, 1-Nal, 2-Nal, h-1-Nal, h-2-Nal, Cha, Chg, Phg;
and salts thereof for use in therapy in combination with an insulin for the treatment of diabetes related ocular pathologies.

2. Peptides for use according to claim 1, selected from Ac-Arg-Glu-Arg-Phe-NH₂; pGlu-Arg-Glu-Arg-Tyr-OH; Glu-Arg-Glu-Arg-Phe-NH₂; Ac-Arg-Glu-Arg-Tyr-NH₂; Ac-Arg-Glu-Arg-Trp-NH₂; Ac-Arg-Glu-N(Me)Arg-Phe-NH₂; Ac-Arg-Glu-N(Me)Arg-Tyr-NH₂; Ac-Arg-Glu-N(Me)Arg-Trp-NH₂; pGlu-Arg-Glu-N(Me)Arg-Phe-NH₂; pGlu-Arg-Glu-N(Me)Arg-Tyr-NH₂; pGlu-Arg-Glu-N(Me)Arg-Trp-NH₂; pGlu-Arg-Glu-Arg-Phe-NH₂; pGlu-Arg-Glu-Arg-Tyr-NH₂; pGlu-Arg-Glu-Arg-Trp-NH₂; Ac-Arg-Aib-Arg-Phe-NH₂; Ac-Arg-Aib-Arg-Tyr-NH₂; Ac-Arg-Aib-Arg-Trp-NH₂; Ac-Aib-Arg-Aib-Arg-Phe-NH₂; Ac-Arg-Aib-N(Me)Arg-Phe-NH₂; Ac-Arg-Aib-N(Me)Arg-Tyr-NH₂; Ac-Arg-Aib-N(Me)Arg-Trp-NH₂; pGlu-Arg-Aib-N(Me)Arg-Phe-NH₂; Glu-Arg-Aib-N(Me)Arg-Tyr-NH₂; pGlu-Arg-Aib-N(Me)Arg-Trp-NH₂; pGlu-Arg-Aib-Arg-Phe-NH₂; pGlu-Arg-Aib-Arg-Tyr-NH₂; pGlu-Arg-Aib-Arg-Trp-NH₂; Ac-Arg-Ac5c-Arg-Phe-NH₂; Ac-Arg-Ac5c-Arg-Tyr-NH₂; Ac-Arg-Ac5c-Arg-Trp-NH₂; Ac-Arg-Ac5c-N(Me)Arg-Phe-NH₂; Ac-Arg-Ac5c-N(Me)Arg-Tyr-NH₂; Ac-Arg-Ac5c-N(Me)Arg-Trp-NH₂; pGlu-Arg-Ac5c-N(Me)Arg-Phe-NH₂; pGlu-Arg-Ac5c-N(Me)Arg-Tyr-NH₂; pGlu-Arg-Ac5c-N(Me)Arg-Trp-NH₂; pGlu-Arg-Ac5c-Arg-Phe-NH₂; pGlu-Arg-Ac5c-Arg-Tyr-NH₂; pGlu-Arg-Ac5c-Arg-Trp-NH₂; Ac-Arg-Glu-Arg-Phe-OH; Ac-Arg-Glu-Arg-Tyr-OH; Ac-Arg-Glu-Arg-Trp-OH; Ac-Arg-Glu-N(Me)Arg-Tyr-OH; pGlu-Arg-Glu-N(Me)Arg-Phe-OH; pGlu-Arg-Glu-Arg-Trp-OH; Ac-Arg-Aib-Arg-Phe-OH; Ac-Arg-Aib-N(Me)Arg-Phe-OH; pGlu-Arg-Aib-N(Me)Arg-Tyr-OH; pGlu-Arg-Aib-Arg-Trp-OH; Ac-Arg-Ac5c-Arg-Phe-OH; Ac-Arg-Ac5c-N(Me)Arg-Tyr-OH; pGlu-Arg-Ac5c-N(Me)Arg-Trp-OH; pGlu-Arg-Ac5c-Arg-Trp-OH; Ac-N(Me)Arg-Aib-Arg-Phe-NH₂; Ac-N(Me)Arg-Aib-N(Me)Arg-Phe-NH₂; Ac-Arg-Aib-N(Me)Arg-Phe-NH₂; Ac-Arg-Aib-Arg-α(Me)Phe-NH₂; Ac-N(Me)Arg-Aib-Arg-(Me)Phe-NH₂; Ac-N(Me)Arg-Aib-N(Me)Arg-α(Me)Phe-NH₂; Ac-Arg-Aib-N(Me)Arg-α(Me)Phe-NH₂; Ac-Aib-N(Me)Arg-Aib-Arg-Phe-NH₂; Ac-Aib-N(Me)Arg-Aib-N(Me)Arg-Phe-NH₂; Ac-Aib-Arg-Aib-N(Me)Arg-Phe-NH₂; Ac-Aib-Arg-Aib-Arg-α(Me)Phe-NH₂; Ac-Aib-N(Me)Arg-Aib-Arg-α(Me)Phe-NH₂; Ac-Aib-N(Me)Arg-Aib-N(Me)Arg-α(Me)Phe-NH₂; Ac-Aib-Arg-Aib-N(Me)Arg-α(Me)Phe-NH₂ or salts thereof.

3. Peptides for use according to claim 1, selected from Ac-Arg-Aib-Arg-α (Me)Phe-NH₂ or Ac-Aib-Arg-Aib-Arg-α(Me)Phe-NH₂ or salts thereof.

4. Pharmaceutical formulations comprising a combination of a peptide as defined in the above claims and insulin in a form suitable for separate, simultaneous or sequential administration.

5. Pharmaceutical formulations according to claim 4 for use in the treatment of diabetes complications, in particular diabetic retinopathy.

6. Pharmaceutical formulations according to claims 4 or 5 comprising a unit dose of the peptide ranging from 1 to 200 mg in combination with standard dosages of insulin.

7. Pharmaceutical formulations according to claims 4 or 5 suitable for administration by the subcutaneous, intramuscular, intravenous, oral, nasal, sublingual, topical, aerosol or trans-dermal route, or by inhalation.

8. Pharmaceutical formulations according to claim 7 suitable for subcutaneous administration.

9. Pharmaceutical formulations according to any one of claims 4-8 comprising a peptide selected from Ac-Arg-Aib-Arg-α(Me)Phe-NH₂ or Ac-Aib-Arg-Aib-Arg-α(Me)Phe-NH₂ or salts thereof.

## Patentansprüche

1. Peptide der allgemeinen Formel (I):
L₁-X₁-X₂-X₃-X₄,
worin:
L₁ für H oder Acyl oder eine gegebenenfalls N-acylierte und/oder N-alkylierte und/oder Cα-alkylierte Aminosäure, ausgewählt aus Glu, Gln, Pro, Hydroxy-Pro, Azt, Pip, pGlu, Aib, Ac4c, Ac5c, Ac6c, steht;
X₁ und X₃, die gleich oder unterschiedlich sein können, eine gegebenenfalls N-alkylierte und/oder Cα-alkylierte basische Aminosäure, ausgewählt aus Arg, Orn und gegebenenfalls guanidyliertem Lys und Phenylalaninen, substituiert an der meta- oder para-Position mit einer Amino- oder Guanidinogruppe, sind;
X₂ eine gegebenenfalls N-alkylierte Aminosäure, ausgewählt aus Glu, Lys, α-Methylleucin, α-Methylvalin, α-Methylglutaminsäure, Aib, Ac4c, Ac5c, Ac6c, ist;
X₄ eine hydrophobe Aminosäure, die an dem C-terminalen Ende amidiert oder nicht-amidiert ist und gegebenenfalls Cα-alkyliert ist, ausgewählt aus Phe, h-Phe, Tyr, Trp, 1-Nal, 2-Nal, h-1-Nal, h-2-Nal, Cha, Chg, Phg, ist;
und Salze davon zur Verwendung in einer Therapie in Kombination mit einem Insulin für die Behandlung von mit Diabetes in Beziehung stehenden Augenkrankheiten.

2. Peptide zur Verwendung gemäß Anspruch 1, ausgewählt aus Ac-Arg-Glu-Arg-Phe-NH₂; pGlu-Arg-Glu-Arg-Tyr-OH; Glu-Arg-Glu-Arg-Phe-NH₂; Ac-Arg-Glu-Arg-Tyr-NH₂; Ac-Arg-Glu-Arg-Trp-NH₂, Ac-Arg-Glu-N(Me)Arg-Phe-NH₂; Ac-Arg-Glu-N(Me)Arg-Tyr-NH₂; Ac-Arg-Glu-N(Me)Arg-Trp-NH₂; pGlu-Arg-Glu-N(Me)Arg-Phe-NH₂; pGlu-Arg-Glu-N(Me)Arg-Tyr-NH₂; pGlu-Arg-Glu-N(Me)Arg-Trp-NH₂; pGlu-Arg-Glu-Arg-Phe-NH₂; pGlu-Arg-Glu-Arg-Tyr-NH₂; pGlu-Arg-Glu-Arg-Trp-NH₂; Ac-Arg-Aib-Arg-Phe-NH₂; Ac-Arg-Aib-Arg-Tyr-NH₂; Ac-Arg-Aib-Arg-Trp-NH₂; Ac-Aib-Arg-Aib-Arg-Phe-NH₂; Ac-Arg-Aib-N(Me)Arg-Phe-NH₂; Ac-Arg-Aib-N(Me)Arg-Tyr-NH₂; Ac-Arg-Aib-N(Me)Arg-Trp-NH₂; pGlu-Arg-Aib-N(Me)Arg-Phe-NH₂; Glu-Arg-Aib-N(Me)Arg-Tyr-NH₂; pGlu-Arg-Aib-N(Me)Arg-Trp-NH₂; pGlu-Arg-Aib-Arg-Phe-NH₂; pGlu-Arg-Aib-Arg-Tyr-NH₂; pGlu-Arg-Aib-Arg-Trp-NH₂; Ac-Arg-Ac5c-Arg-Phe-NH₂; Ac-Arg-Ac5c-Arg-Tyr-NH₂; Ac-Arg-Ac5c-Arg-Trp-NH₂; Ac-Arg-Ac5c-N(Me)Arg-Phe-NH₂; Ac-Arg-Ac5c-N(Me)Arg-Tyr-NH₂; Ac-Arg-Ac5c-N(Me)Arg-Trp-NH₂; pGlu-Arg-Ac5c-N(Me)Arg-Phe-NH₂; pGlu-Arg-Ac5c-N(Me)Arg-Tyr-NH₂; pGlu-Arg-Ac5c-N(Me)Arg-Trp-NH₂; pGlu-Arg-Ac5c-Arg-Phe-NH₂; pGlu-Arg-Ac5c-Arg-Tyr-NH₂; pGlu-Arg-Ac5c-Arg-Trp-NH₂; Ac-Arg-Glu-Arg-Phe-OH; Ac-Arg-Glu-Arg-Tyr-OH; Ac-Arg-Glu-Arg-Trp-OH; Ac-Arg-Glu-N(Me)Arg-Tyr-OH; pGlu-Arg-Glu-N(Me)Arg-Phe-OH; pGlu-Arg-Glu-Arg-Trp-OH; Ac-Arg-Aib-Arg-Phe-OH; Ac-Arg-Aib-N(Me)Arg-Phe-OH; pGlu-Arg-Aib-N(Me)Arg-Tyr-OH; pGlu-Arg-Aib-Arg-Trp-OH; Ac-Arg-Ac5c-Arg-Phe-OH; Ac-Arg-Ac5c-N(Me)Arg-Tyr-OH; pGlu-Arg-Ac5c-N(Me)Arg-Trp-OH; pGlu-Arg-Ac5c-Arg-Trp-OH; Ac-N(Me)Arg-Aib-Arg-Phe-NH₂; Ac-N(Me)Arg-Aib-N(Me)Arg-Phe-NH₂; Ac-Arg-Aib-N(Me)Arg-Phe-NH₂; Ac-Arg-Aib-Arg-α(Me)Phe-NH₂; Ac-N(Me)Arg-Aib-Arg-(Me)Phe-NH₂; Ac-N(Me)Arg-Aib-N(Me)Arg-α(Me)Phe-NH₂; Ac-Arg-Aib-N(Me)Arg-α(Me)Phe-NH₂; Ac-Aib-N(Me)Arg-Aib-Arg-Phe-NH₂; Ac-Aib-N(Me)Arg-Aib-N(Me)Arg-Phe-NH₂; Ac-Aib-Arg-Aib-N(Me)Arg-Phe-NH₂; Ac-Aib-Arg-Aib-Arg-α(Me)Phe-NH₂; Ac-Aib-N(Me)Arg-Aib-Arg-a(Me)Phe-NH₂; Ac-Aib-N(Me)Arg-Aib-N(Me)Arg-α(Me)Phe-NH₂; Ac-Aib-Arg-Aib-N(Me)Arg-α(Me)Phe-NH₂ oder Salze davon.

3. Peptide zur Verwendung gemäß Anspruch 1, ausgewählt aus Ac-Arg-Aib-Arg-α(Me)Phe-NH₂ oder Ac-Aib-Arg-Aib-Arg-α(Me)Phe-NH₂, oder Salze davon.

4. Pharmazeutische Formulierungen, die eine Kombination aus einem Peptid, wie in den obigen Ansprüchen definiert, und Insulin umfassen, in einer Form, die zur getrennten, gleichzeitigen oder aufeinanderfolgenden Verabreichung geeignet ist.

5. Pharmazeutische Formulierungen gemäß Anspruch 4 zur Verwendung in der Behandlung von Diabetes-Komplikationen, insbesondere diabetischer Retinopathie.

6. Pharmazeutische Formulierungen gemäß Anspruch 4 oder 5, die eine Dosiseinheit des Peptids im Bereich von 1 bis 200 mg in Kombination mit Standarddosierungen von Insulin umfassen.

7. Pharmazeutische Formulierungen gemäß Anspruch 4 oder 5, die zur Verabreichung auf dem subkutanen, intramuskulären, intravenösen, oralen, nasalen, sublingualen, topischen, Aerosol- oder transdermalen Weg oder durch Inhalation geeignet sind.

8. Pharmazeutische Formulierungen gemäß Anspruch 7, die zur subkutanen Verabreichung geeignet sind.

9. Pharmazeutische Formulierungen gemäß einem beliebigen der Ansprüche 4 bis 8, umfassend ein Peptid, ausgewählt aus Ac-Arg-Aib-Arg-α(Me)Phe-NH₂ oder Ac-Aib-Arg-Aib-Arg-α(Me)Phe-NH₂ oder Salzen davon.

## Revendications

1. Peptides répondant à la formule générale (I) :
L₁-X₁-X₂-X₃-X₄,
dans laquelle
L₁ représente un atome d'hydrogène ou un groupe acyle ou encore un acide aminé de manière facultative acylé sur l'atome d'azote et/ou alkylé sur l'atome d'azote et/ou alkylé sur l'atome de carbone alpha, choisi parmi Glu, Gin, Pro, hydroxy-Pro, Azt, Pip, pGlu, Aib, Ac4c, Ac5c, Ac6c ;
X₁ et X₃, qui peuvent être identiques ou différents, représentent un acide aminé basique de manière facultative alkylé sur l'atome d'azote et/ou alkylé sur l'atome de carbone alpha, choisi parmi Arg, Orn et Lys de manière facultative guanidylé, et des phénylalanines substituées aux positions *méta* ou *para* avec un groupe amino ou un groupe guanidino ;
X₂ représente un acide aminé de manière facultative alkylé sur l'atome d'azote, choisi parmi Glu, Lys, l'α-méthyl-leucine, l'a-méthyl-valine, l'acide α-méthyl-glutamique, Aib, Ac4c, Ac5c, Ac6c ;
X₄ représente un acide aminé hydrophobe qui est amidé ou non amidé à l'extrémité carboxy-terminale et de manière facultative alkylé sur l'atome de carbone alpha, choisi parmi Phe, h-Phe, Tyr, Trp, 1-Nal, 2-Nal, h-1-Nal, h-2-Nal, Cha, Chg, Phg ;
ainsi que leurs sels pour leur utilisation dans une thérapie en combinaison avec une insuline pour le traitement de pathologies oculaires liées au diabète.

2. Peptides pour leur utilisation selon la revendication 1, choisis parmi Ac-Arg-Glu-Arg-Phe-NH₂; pGlu-Arg-Glu-Arg-Tyr-OH; Glu-Arg-Glu-Arg-Phe-NH₂; Ac-Arg-Glu-Arg-Tyr-NH₂; Ac-Arg-Glu-Arg-Trp-NH₂; Ac-Arg-Glu-N(Me)Arg-Phe-NH₂; Ac-Arg-Glu-N(Me)Arg-Tyr-NH₂; Ac-Arg-Glu-N(Me)Arg-Trp-NH₂; pGlu-Arg-Glu-N(Me)Arg-Phe-NH₂; pGlu-Arg-Glu-N(Me)Arg-Tyr-NH₂; pGlu-Arg-Glu-N(Me)Arg-Trp-NH₂; pGlu-Arg-Glu-Arg-Phe-NH₂; pGlu-Arg-Glu-Arg-Tyr-NH₂; pGlu-Arg-Glu-Arg-Trp-Nh₂; Ac-Arg-Aib-Arg-Phe-NH₂; Ac-Arg-Aib-Arg-Tyr-NH₂; Ac-Arg-Aib-Arg-Trp-NH₂; Ac-Aib-Arg-Aib-Arg-Phe-NH₂; Ac-Arg-Aib-N(Me)Arg-Phe-NH₂; Ac-Arg-Aib-N(Me)Arg-Tyr-NH₂; Ac-Arg-Aib-N(Me)Arg-Trp-NH₂; pGlu-Arg-Aib-N(Me)Arg-Phe-NH₂; Glu-Arg-Aib-N(Me)Arg-Tyr-NH₂; pGlu-Arg-Aib-N(Me)Arg-Trp-NH₂; pGlu-Arg-Aib-Arg-Phe-NH₂; pGlu-Arg-Aib-Arg-Tyr-NH₂; pGlu-Arg-Aib-Arg-Trp-NH₂; Ac-Arg-Ac5c-Arg-Phe-NH₂; Ac-Arg-Ac5c-Arg-Tyr-NH₂; Ac-Arg-Ac5c-Arg-Trp-NH₂; Ac-Arg-Ac5c-N(Me)Arg-Phe-NH₂; Ac-Arg-Ac5c-N(Me)Arg-Tyr-NH₂; Ac-Arg-Ac5c-N(Me)Arg-Trp-NH₂; pGlu-Arg-Ac5c-N(Me)Arg-Phe-NH₂; pGlu-Arg-Ac5c-N(Me)Arg-Tyr-NH₂; pGlu-Arg-Ac5c-N(Me)Arg-Trp-NH₂; pGlu-Arg-Ac5c-Arg-Phe-NH₂; pGlu-Arg-Ac5c-Arg-Tyr-NH₂; pGlu-Arg-Ac5c-Arg-Trp-NH₂; Ac-Arg-Glu-Arg-Phe-OH; Ac-Arg-Glu-Arg-Tyr-OH; Ac-Arg-Glu-Arg-Trp-OH; Ac-Arg-Glu-N(Me)Arg-Tyr-OH; pGlu-Arg-Glu-N(Me)Arg-Phe-OH; pGlu-Arg-Glu-Arg-Trp-OH; Ac-Arg-Aib-Arg-Phe-OH; Ac-Arg-Aib-N(Me)Arg-Phe-OH; pGlu-Arg-Aib-N(Me)Arg-Tyr-OH; pGlu-Arg-Aib-Arg-Trp-OH; Ac-Arg-Ac5c-Arg-Phe-OH; Ac-Arg-Ac5c-N(Me)Arg-Tyr-OH; pGlu-Arg-Ac5c-N(Me)Arg-Trp-OH; pGlu-Arg-Ac5c-Arg-Trp-OH; Ac-N(Me)Arg-Aib-Arg-Phe-NH₂; Ac-N(Me)Arg-Aib-N(Me)Arg-Phe-NH₂; Ac-Arg-Aib-N(Me)Arg-Phe-NH₂; Ac-Arg-Aib-Arg-α(Me)Phe-NH₂; Ac-N(Me)Arg-Aib-Arg-α(Me)Phe-NH₂; Ac-N(Me)Arg-Aib-N(Me)Arg-α(Me)Phe-NH₂; Ac-Arg-Aib-N(Me)Arg-α(Me)Phe-NH₂; Ac-Aib-N(Me)Arg-Aib-Arg-Phe-NH₂; Ac-Aib-N(Me)Arg-Aib-N(Me)Arg-Phe-NH₂; Ac-Aib-Arg-Aib-N(Me)Arg-Phe-NH₂; Ac-Aib-Arg-Aib-Arg-α(Me)Phe-NH₂; Ac-Aib-N(Me)Arg-Aib-Arg-α(Me)Phe-NH₂; Ac-Aib-N(Me)Arg-Aib-N(Me)Arg-α(Me)Phe-NH₂; Ac-Aib-Arg-Aib-N(Me)Arg-α(Me)Phe-NH₂ ou leurs sels.

3. Peptides pour leur utilisation selon la revendication 1, choisis parmi Ac-Arg-Aib-Arg-α(Me)Phe-NH₂ ou Ac-Aib-Arg-Aib-Arg-α(Me)Phe-NH₂ ou leurs sels.

4. Formulations pharmaceutiques comprenant une combinaison d'un peptide tel que défini dans les revendications ci-dessus et d'insuline sous une forme appropriée pour une administration séparée, simultanée ou séquentielle.

5. Formulations pharmaceutiques selon la revendication 4, pour leur utilisation dans le traitement de complications diabétiques, en particulier de la rétinopathie diabétique.

6. Formulations pharmaceutiques selon la revendication 4 ou 5, comprenant une dose unitaire du peptide qui se situe dans une plage de 1 à 200 mg en combinaison avec des posologies standard d'insuline.

7. Formulations pharmaceutiques selon la revendication 4 ou 5, appropriées pour une administration par la voie sous-cutanée, intramusculaire, intraveineuse, orale, nasale, sublinguale, locale, par aérosol ou transdermique, ou encore par inhalation.

8. Formulations pharmaceutiques selon la revendication 7, appropriées pour une administration par voie sous-cutanée.

9. Formulations pharmaceutiques selon l'une quelconque des revendications 4 à 8, comprenant un peptide choisi parmi Ac-Arg-Aib-Arg-α(Me)Phe-NH₂ ou Ac-Aib-Arg-Aib-Arg-α(Me)Phe-NH₂ ou leurs sels.
